# EUROPÄISCHE PATENTSCHRIFT

(11) **EP 0 763 347 B1**
(45) Veröffentlichungstag und Bekanntmachung des Hinweises auf die Patenterteilung: **28.10.1998**
(21) Anmeldenummer: 96112775.0
(22) Anmeldetag: 08.08.1996
(51) Int. Cl.: A61B 17/122

(54) **Chirurgischer Gefässclip**
Surgical blood vessel clip
Pince chirurgicale hémostatique

(30) Priorität: 15.09.1995 DE 19534322
(43) Veröffentlichungstag der Anmeldung: 19.03.1997
(73) Patentinhaber: AESCULAP AG, 78532 Tuttlingen (DE)
(72) Erfinder: Back, Lothar, D-72514 Inzighofen (DE); Herrmann, Gebhard, D-78597 Irndorf (DE); Nesper, Markus, D-78532 Tuttlingen (DE); Weeisshaupt, Dieter, D-78194 Immendingen (DE)
(74) Vertreter: Böhme, Ulrich, Dr. Dipl.-Phys.

(56) Entgegenhaltungen:
- WO-A-92/19144
- DE-A- 3 327 721
- GB-A- 2 025 511
- US-A- 4 337 774
- US-A- 4 733 666

## Beschreibung

Die Erfindung betrifft einen chirurgischen Gefäßclip mit zwei federnd gegeneinanderdrückbaren, Klemmbacken aufweisenden Armen, die an einem Endabschnitt des Clips zusammentreffen, und mit einem sich in Richtung auf die Klemmbacken an den Endabschnitt anschließenden Spannabschnitt, der von einem in Längsrichtung des Spannabschnittes verschiebbaren Ring umgeben ist, wobei die Arme mit den Klemmbacken, der Spannabschnitt und der Endabschnitt als einteiliges Bauteil ausgebildet sind.

Es ist möglich, derartige Clips mit einem speziellen Applikator an die abzuklemmenden Gefäßteile anzulegen, wobei der Clip durch die Verschiebung des Ringes auf dem Spannabschnitt geschlossen werden kann.

Bei bekannten Clips besteht dieser aus Halbrundprofilstäben, die im Bereich des Endabschnittes aneinander anliegen und dort miteinander verbunden werden müssen, beispielsweise durch Verschweißen oder durch Verpressen. Da es sich bei diesen Clips um sehr kleine Teile handelt, ist eine solche Verbindungstechnik schwierig zu bewerkstelligen.

Es ist bei chirurgischen Nahtinstrumenten, also bei Vorrichtungen zur Herstellung einer nahtähnlichen Verbindung, bekannt, zweiarmige Verbindungselemente mit einem längsverschieblichen Ring einteilig auszubilden, es handelt sich dabei aber ausschließlich um Teile, die nach Art einer schlaufenförmigen Nahtverbindung ineinandergreifen sollen, die also keinerlei Klemmfunktion haben, wie dies bei einem chirurgischen Clip notwendig ist. Gerade bei einem chirurgischen Gefäßclip sind hohe Klemmkräfte notwendig, und daher muß sichergestellt sein, daß die Klemmbacken unter hoher Spannkraft dauerhaft gegeneinander gepreßt werden. Daher sind derartige chirurgische Gefäßclips mit Nahtvorrichtungen der erörterten Art nicht vergleichbar (US-A-5,366,459).

In der DE 33 27 721 A1 ist ein einteiliger Clip beschrieben, der zwei im Querschnitt quadratische Arme aufweist, die über ein Scharniergelenk miteinander verbunden sind. Dieses kompliziert die Herstellung.

Es ist Aufgabe der Erfindung, einen chirurgischen Clip der gattungsgemäßen Art so auszubilden, daß die Herstellung des Clips vereinfacht werden kann, insbesondere ohne aufwendige Verbindungen im Endabschnittsbereich zu benötigen.

Diese Aufgabe wird bei einem Clip der eingangs beschriebenen Art erfindungsgemäß dadurch gelöst, daß er aus einem länglichen Rotationskörper besteht, der durch einen bis an den Endabschnitt heranreichenden Längsschlitz in zwei Arme unterteilt ist.

Bei der Herstellung wird also zunächst beispielsweise auf einer Drehbank ein Rotationskörper hergestellt, und dieser Rotationskörper wird ausgehend von der dem Endabschnitt gegenüberliegenden Spitze durch einen Längsschlitz in zwei Arme oder Schenkel aufgetrennt. Es ist damit in einfacher Weise möglich, einen einstückigen Clip herzustellen, ohne aufwendige Verbindungen im Endabschnittsbereich zu benötigen.

Günstig ist es, wenn der Endabschnitt die Form eines Kreiszylinders aufweist, der stufig über den anschließenden Spannabschnitt vorspringt. Dieser Endabschnitt kann verwendet werden, um den Clip in einem Applikator einzuspannen, so daß der Applikator den Clip zuverlässig hält und außerdem den auf ihm angeordneten Ring verschieben kann.

Günstig ist es, wenn der Spannabschnitt zu den Klemmbacken hin durch einen Vorsprung abgeschlossen wird, der vorzugsweise die Form einer die Arme außenseitig umgebenden Schulter hat. Durch einen solchen Vorsprung wird die Verschiebemöglichkeit des Ringes auf dem Spannabschnitt begrenzt, es besteht daher keine Gefahr, daß der Ring zu weit geschoben wird.

Es ist auch möglich, daß der Spannabschnitt zu dem Endabschnitt hin durch einen Vorsprung abgeschlossen wird, auch dieser hat vorzugsweise die Form einer die Arme außenseitig umgebenden Schulter. Auf diese Weise wird die Verschiebebewegung des Ringes längs des Spannabschnittes auf den Bereich zwischen diesen Vorsprüngen begrenzt. Dies ist insbesondere dann nützlich, wenn der Ring nach innen zwischen die Arme vorspringende Vorsprünge trägt und durch diese Vorsprünge die Arme beim Zurückschieben aufbiegt. Diese Aufbiegung wird durch den Vorsprung zum Endabschnitt des Clips hin begrenzt.

Es kann vorgesehen sein, daß sich der Spannabschnitt vom Endabschnitt zu den Klemmbacken hin kegelig erweitert.

Bei einer anderen Ausführungsform ist vorgesehen, daß sich an den sich kegelig erweiternden Teil des Spannabschnittes noch ein zylindrischer Teil anschließt. Beim Vorschieben des Ringes erfolgt im kegeligen Teil ein zunehmendes Schließen des Clips, im zylindrischen Teil hingegen bleibt die Spannung erhalten, so daß durch ein Verschieben in diesem Bereich einmal eine gewisse Toleranz für die Verschiebung des Ringes erreicht wird, zum anderen wird dadurch sichergestellt, daß der Ring nicht ungewollt in die Offenstellung zurückgleitet.

Bei einer weiteren bevorzugten Ausführungsform ist vorgesehen, daß die Arme im Bereich des Spannabschnittes in Längsrichtung gewellt sind, es sind also Querrippen um den Clip gelegt, so daß der Ring durch diese Wellung oder Querrippen festgehalten und am Abgleiten längs des Spannabschnittes gehindert wird. Der Ring kann dazu zusätzlich an seiner Innenseite in die Wellung beziehungsweise in die Rippen eingreifende Vorsprünge tragen. Dadurch ergibt sich auch eine stufige Verschiebung des Ringes längs des Spannabschnittes und damit eine stufige Spannungserzeugung im Bereich der Klemmbacken, der Operateur hat dadurch die Möglichkeit, genau definiert unterschiedliche Spannungswerte einzustellen.

Günstig ist es, wenn die Arme im Spannabschnitt zwischen sich einen Spalt einschließen, dessen Breite sich vom Endabschnitt zu den Klemmbacken vergrößert. In diesen Spalt können Vorsprünge des Ringes eingreifen, die beim Zurückschieben des Ringes zu einem Spreizen der Klemmbacken führen.

Dabei ist es weiterhin vorteilhaft, wenn der Spalt am klemmbackenseitigen Ende des Spannabschnittes zum anderen Arm hin gerichtete Endflächen aufweist. Damit wird der sich erweiternde Spalt zu den Klemmbacken hin wieder verengt.

Bei einer besonders bevorzugten Ausführungsform ist vorgesehen, daß die Arme zwischen den Klemmbacken und dem Spannabschnitt bogenförmig nach außen gebogen sind. Es entsteht dadurch eine Öffnung in diesem Zwischenbereich, durch den beispielsweise ein Gefäß hindurchlaufen kann. Es ist dadurch möglich, den Clip auch in Bereichen einzusetzen, in denen Körperteile verlaufen, die nicht abgeklemmt werden sollen.

Bei einem Clip mit bogenförmig nach außen gebogenen Armen kann vorgesehen sein, daß die bogenförmig nach außen gebogenen Bereiche der Arme aus einer bauchigen Verdickung des Rotationskörpers durch beidseitige Abflachung und durch eine bauchige Erweiterung des Längsschlitzes gebildet werden. Ausgehend von einem Rotationskörper wird dieser mit einer bauchigen Verdickung hergestellt, beispielsweise mit einer kugelförmigen Verdickung, diese wird anschließend beispielsweise durch Fräsen beidseitig abgeflacht und in diesem dann entstehenden scheibenförmigen Bereich des Rotationskörpers wird der Längsschlitz bauchig verbreitert, so daß die Kontur des Längsschlitzes im wesentlichen parallel zur Außenkontur der Scheibe verläuft. Dadurch lassen sich bogenförmige Armabschnitte herstellen, die den gewünschten Fensterbereich erzeugen.

Vorteilhaft ist es auch, wenn der Längsschlitz im Bereich der Klemmbacken im wesentlichen eine konstante Breite aufweist, sich beim Eintritt in den Spannabschnitt erweitert und in Richtung auf den Endabschnitt in seiner Breite wieder abnimmt. Beim Zusammenbiegen der Klemmbacken werden sich diese aufgrund der einstükkigen Verbindung im Bereich des Endabschnittes so einander annähern, daß sie sich zunächst am freien Ende berühren und dann fortlaufend von vorne nach hinten zur Anlage kommen. Dies stellt sicher, daß die Klemmbacken nicht nach vorne hin aufklaffen, sondern immer zuverlässig geschlossen sind. Dieser Effekt kann im übrigen unterstützt werden, wenn der Schlitz im Bereich der Klemmbacken sich in Richtung auf den Endabschnitt hin geringfügig erweitert.

Der Längsschlitz läßt sich besonders vorteilhaft mit einer Drahterodiermaschine erzeugen, es können aber auch andere Herstellungsverfahren verwendet werden, beispielsweise das Verfahren mittels eines Elektronenstrahles oder das Auftrennen mittels eines Laserstrahles.

Die nachfolgende Beschreibung bevorzugter Ausführungsformen der Erfindung dient im Zusammenhang mit der Zeichnung der näheren Erläuterung. Es zeigen:
- Figur 1:: einen Clip mit gewelltem, kegeligem Spannabschnitt;
- Figur 2:: einen Clip mit glattem, kegeligem Spannabschnitt;
- Figur 3 :: einen Clip mit einem Spannabschnitt mit kegeligen und zylindrischen Bereichen;
- Figur 4:: einen Clip mit bogenförmig gebogenen Armen zur Ausbildung eines Fensters und
- Figur 5:: einen Clip mit einem schulterförmigen Vorsprung zur endabschnittseitigen Begrenzung des Spannabschnittes.

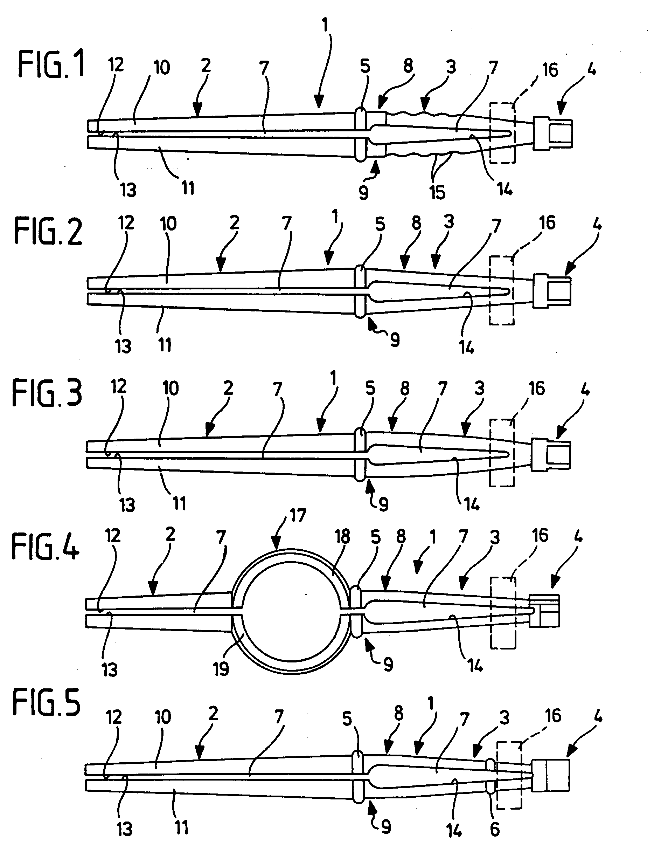

Alle in den Figuren 1 bis 5 dargestellten Clips 1 werden als längliche Rotations- oder Drehkörper hergestellt, beispielsweise auf einer Drehbank. Diese Rotationskörper weisen einen sich geringfügig konisch erweiternden Klemmaschnitt 2, einen sich daran anschliessenden, im Außendurchmesser abnehmenden Spannabschnitt 3 und einen gegenüber diesem stufig vorspringenden, zylindrischen Endabschnitt 4 auf. Der Endabschnitt 4 kann als massiver Zylinder ausgebildet sein oder als zur Rückseite hin offener, hülsenförmiger Zylinder, der gegebenenfalls auch einen stufigen Rücksprung aufweisen kann.

Zwischen dem Klemmabschnitt 2 und dem Spannabschnitt 3 ist bei allen Ausführungsbeispielen ein Vorsprung in Form einer den Rotationskörper umgebenden Ringschulter 5 vorgesehen, eine weitere Ringschulter 6 kann bei einem bevorzugten Ausführungsbeispiel (Figur 5) auch noch im rückwärtigen Teil des Spannabschnittes 3 in der Nähe des Endabschnittes 4 vorgesehen werden.

Dieser Rotationskörper wird in allen Fällen durch einen Längsschlitz 7 in zwei Arme 8, 9 unterteilt, die im Bereich des Endabschnittes 4 einstückig miteinander verbunden sind. Dieser Längsschlitz 7 hat im Klemmabschnitt 2 eine konstante und geringe Breite, so daß der Klemmabschnitt 2 in zwei Klemmbacken 10, 11 unterteilt wird, die mit ebenen Klemmflächen 12, 13 einander gegenüberliegen. Diese Klemmflächen 12, 13 können profiliert sein, beispielsweise können sie eine Prägung erhalten.

Der Längsschlitz 7 erweitert sich beim Eintritt in den Spannabschnitt 3 und verengt sich in Richtung auf den Endabschnitt 4, so daß im Bereich des Spannabschnittes 3 ein keilförmiger Zwischenraum 14 entsteht.

Der Längsschlitz 7 kann durch verschiedene Verfahren hergestellt werden, besonders vorteilhaft ist die Herstellung in einem Drahterodierverfahren oder mit Hilfe eines Strahlverfahrens, beispielsweise mit Hilfe eines Elektronenstrahles oder eines Laserstrahles. Durch all diese Verfahren kann ein Längsschlitz 7 mit einer bestimmten Kontur in den Rotationskörper eingeschnitten werden.

Im Bereich des Spannabschnittes 3 sind unterschiedliche Außenkonturen möglich. Bei dem Ausführungsbeispiel der Figur 2 ist die Außenkontur in diesem Bereich über die gesamte Länge des Spannabschnittes kegelig, bei dem Ausführungsbeispiel der Figur 3 schließt sich an den kegeligen Teil des Spannabschnittes ein zylindrischer Teil an. Beim Ausführungsbeispiel der Figur 1 ist der Spannabschnitt ebenfalls kegelig ausgebildet, zusätzlich ist die Außenkontur in Längsrichtung wellig geformt, so daß sich in Umfangsrichtung umlaufende Rippen 15 ausbilden.

Auf dem Spannabschnitt 3 wird bei allen Clips 1 ein Ring 16 aufgesteckt, der die beiden Arme 8, 9 umgibt und längs des Spannabschnittes verschiebbar ist. Dieser Ring 16 wird als separates Teil ausgebildet und beispielsweise durch Zusammenpressen der Arme 8, 9 über die Ringschulter 5 gesteckt. Dieser Ring kann nach innen vorstehende Vorsprünge tragen, die in den keilförmigen Zwischenraum 14 eingreifen und beim Zurückschieben des Ringes die Arme 8, 9 aufspreizen. Beim Vorschieben des Ringes drückt dieser die beiden Arme 8 und 9 elastisch zusammen und preßt dadurch die Klemmflächen 12, 13 gegeneinander.

Bei dem Ausführungsbeispiel der Figur 4 ist zwischen den Klemmbacken 10, 11 einerseits und dem Spannabschnitt 3 andererseits noch ein Fensterabschnitt 17 angeordnet. In diesem Bereich sind die Arme 8, 9 bogenförmig nach außen geformt und umschließen dadurch eine Fensteröffnung zur Aufnahme von nicht zusammengeklemmten Gewebeteilen.

Diese bogenförmigen Armabschnitte 18, 19 können ebenfalls ausgehend von einem Rotationskörper hergestellt werden. Dazu wird im Bereich des Fensterabschnittes 17 der Rotationskörper mit einer kugeligen Verdickung hergestellt. Diese Verdickung wird dann von zwei Seiten her abgeflacht, beispielsweise durch Abfräsen, und zwar senkrecht zur Ebene des Längsschlitzes 7. Damit wird die kugelige Verdickung zu einer scheibenförmigen Verdickung reduziert. Im Bereich dieser scheibenförmigen Verdickung wird dann der Längsschlitz 7 kreisförmig erweitert, so daß nur noch die bogenförmigen Armabschnitte 18 und 19 stehenbleiben.

Trotz dieser sehr komplizierten Form des Clips ist es damit möglich, einen einteiligen Clip herzustellen, bei dem komplizierte Verbindungstechniken vermieden werden können.

## Patentansprüche

1. Chirurgischer Gefäßclip mit zwei federnd gegeneinanderdrückbaren, Klemmbacken (10, 11) aufweisenden Armen (8, 9), die an einem Endabschnitt (4) des Clips zusammentreffen, und mit einem sich in Richtung auf die Klemmbacken (10, 11) an den Endabschnitt (4) anschließenden Spannabschnitt (3), der von einem in Längsrichtung des Spannabschnittes (3) verschiebbaren Ring (16) umgeben ist, wobei die Arme (8, 9) mit den Klemmbacken (10, 11), der Spannabschnitt (3) und der Endabschnitt (4) als einteiliges Bauteil ausgebildet sind, dadurch gekennzeichnet, daß der Clip aus einem länglichen Rotationskörper besteht, der durch einen bis an den Endabschnitt (4) heranreichenden Längsschlitz (7) in zwei Arme (8, 9) unterteilt ist.

2. Clip nach Anspruch 1, dadurch gekennzeichnet, daß der Endabschnitt (4) die Form eines Kreiszylinders aufweist, der stufig über den anschließenden Spannabschnitt (3) vorspringt.

3. Clip nach Anspruch 1 oder 2, dadurch gekennzeichnet, daß der Spannabschnitt (3) zu den Klemmbacken (10, 11) hin durch einen Vorsprung (5) abgeschlossen wird.

4. Clip nach Anspruch 3, dadurch gekennzeichnet, daß der Vorsprung (5) die Form einer die Arme (8, 9) außenseitig umgebenden Schulter hat.

5. Clip nach einem der Ansprüche 1 bis 3, dadurch gekennzeichnet, daß der Spannabschnitt (3) zu dem Endabschnitt (4) hin durch einen Vorsprung (6) abgeschlossen wird.

6. Clip nach Anspruch 5, dadurch gekennzeichnet, daß der Vorsprung (6) die Form einer die Arme (8, 9) außenseitig umgebenden Schulter hat.

7. Clip nach einem der voranstehenden Ansprüche, dadurch gekennzeichnet, daß sich der Spannabschnitt (3) vom Endabschnitt (4) zu den Klemmbacken (10, 11) hin kegelig erweitert.

8. Clip nach Anspruch 7, dadurch gekennzeichnet, daß sich an den kegelig erweiternden Teil des Spannabschnittes (3) ein zylindrischer Teil anschließt.

9. Clip nach einem der voranstehenden Ansprüche, dadurch gekennzeichnet, daß die Arme (8, 9) im Bereich des Spannabschnittes (3) in Längsrichtung gewellt sind.

10. Clip nach einem der voranstehenden Ansprüche, dadurch gekennzeichnet, daß die Arme (8, 9) im Spannabschnitt (3) zwischen sich einen Spalt (7) einschließen, dessen Breite sich vom Endabschnitt (4) zu den Klemmbacken (10, 11) vergrößert.

11. Clip nach Anspruch 10, dadurch gekennzeichnet, daß der Spalt (7) am klemmbackenseitigen Ende des Spannabschnittes (3) zum anderen Arm hin gerichtete Endflächen aufweist.

12. Clip nach einem der voranstehenden Ansprüche, dadurch gekennzeichnet, daß die Arme (8, 9) zwischen den Klemmbacken (10, 11) und dem Spannabschnitt (3) bogenförmig nach außen gebogen sind.

13. Clip nach Anspruch 12, dadurch gekennzeichnet, daß die bogenförmig nach außen gebogenen Bereiche (18, 19) der Arme (8, 9) aus einer bauchigen Verdickung des Rotationskörpers durch beidseitige Abflachung und durch eine bauchige Erweiterung des Längsschlitzes (7) gebildet werden.

14. Clip nach einem der voranstehenden Ansprüche, dadurch gekennzeichnet, daß der Längsschlitz (7) im Bereich der Klemmbacken (10, 11) im wesentlichen eine konstante Breite aufweist, sich beim Eintritt in den Spannabschnitt (3) erweitert und in Richtung auf den Endabschnitt (4) in seiner Breite wieder abnimmt.

15. Clip nach einem der voranstehenden Ansprüche, dadurch gekennzeichnet, daß der Längsschlitz (7) mit einer Drahterodiermaschine erzeugbar ist.

## Claims

1. A surgical vessel-clip with two arms (8, 9) having clamp jaws (10, 11), resiliently pressable against each other, which arms meet at an end section (4) of the clip, and with a clamping section (3) adjoining the end section (4) in the direction of the clamp jaws (10, 11), which clamping section (3) is encircled by a ring (16) displaceable in a longitudinal direction of the clamping section (3), the arms (8, 9) with the clamp jaws (10, 11), the clamping section (3) and the end section (4) being formed as a one-piece member, characterised in that the clip comprises an elongate body of rotation which is divided into two arms (8, 9) by a longitudinal slot (7) reaching up to the end section (4).

2. A clip according to claim 1, characterised in that the end section (4) has the form of a circular cylinder which projects stepwise beyond the adjoining clamping section (3).

3. A clip according to claim 1 or 2, characterised in that the clamping section (3) is terminated towards the clamp jaws (10, 11) by a projection (5).

4. A clip according to claim 3, characterised in that the projection (5) has the form of a shoulder encircling the arms (8, 9) on the outer side.

5. A clip according to any one of claims 1 to 3, characterised in that the clamping section (3) is terminated towards the end section (4) by a projection (6).

6. A clip according to claim 5, characterised in that the projection (6) has the form of a shoulder encircling the arms (8, 9) on the outer side.

7. A clip according to any one of the preceding claims, characterised in that the clamping section (3) widens conically from the end section (4) towards the clamp jaws (10, 11).

8. A clip according to claim 7, characterised in that a cylindrical part adjoins the conically widening part of the clamping section (3).

9. A clip according to any one of the preceding claims, characterised in that the arms (8, 9) are corrugated in the longitudinal direction in the region of the clamping section (3).

10. A clip according to any one of the preceding claims, characterised in that the arms (8, 9), in the clamping section (3), contain between them a gap (7) whose width increases from the end section (4) towards the clamp jaws (10, 11).

11. A clip according to claim 10, characterised in that the gap (7) at the clamp-jaws-side end of the clamping section (3) has end faces directed towards the other arm.

12. A clip according to any one of the preceding claims, characterised in that the arms (8, 9) are curved outwards in an arch-shape between the clamp jaws (10, 11) and the clamping section (3).

13. A clip according to claim 12, characterised in that the arch-shaped, out-curved regions (18, 19) of the arms (8, 9) are formed from a bulged thickening of the body of rotation, through flattening at both sides and through bulged widening of the longitudinal slot (7).

14. A clip according to any one of the preceding claims, characterised in that the longitudinal slot (7), in the region of the clamp jaws (10, 11), substantially has a constant width, the longitudinal slot (7) widens on entry into the clamping section (3) and its width decreases again in the direction of the end section (4).

15. A clip according to any one of the preceding claims, characterised in that the longitudinal slot (7) can be made by an electroerosion machine.

## Revendications

1. Pince chirurgicale hémostatique comportant deux bras (8, 9) qui présentent des mors de pincement (10, 11) pouvant s'appuyer élastiquement l'un contre l'autre et se rencontrent en une portion d'extrémité (4) de la pince, et comportant une portion de serrage (3) qui se raccorde à la portion d'extrémité (4) en direction des mors de pincement (10, 11) et qui est entourée d'une bague (16) que l'on peut faire coulisser selon la direction longitudinale de la portion de serrage (3), les bras (8, 9), avec les mors de pincement (10, 11), la portion de serrage (3) et la portion d'extrémité (4) étant confus sous forme d'un composant monobloc, caractérisée par le fait que la pince est constituée d'un corps longitudinal de révolution qui est divisé en deux bras (8, 9) par une fente longitudinale (7) qui va jusqu'à la portion d'extrémité (4).

2. Pince selon la revendication 1, caractérisée par le fait que la portion d'extrémité (4) présente la forme d'un cylindre circulaire qui déborde, par échelons, au-delà de la portion de serrage (3) qui s'y raccorde.

3. Pince selon la revendication 1 ou 2, caractérisée par le fait qu'en direction des mors de pincement (10, 11), la portion de serrage (3) se termine par un collet (5).

4. Pince selon la revendication 3, caractérisée par le fait que le collet (5) a la forme d'un épaulement entourant les bras (8, 9) à l'extérieur.

5. Pince selon les revendications 1 à 3, caractérisée par le fait qu'en direction de la portion d'extrémité (4), la portion de serrage (3) se termine par un collet (6).

6. Pince selon la revendication 5, caractérisée par le fait que le collet (6) a la forme d'un épaulement entourant les bras (8, 9) à l'extérieur.

7. Pince selon l'une des revendications précédentes, caractérisée par le fait que la portion de serrage (3) va en s'élargissant en cône, de la portion d'extrémité (4) jusqu'aux mors de pincement (10, 11).

8. Pince selon la revendication 7, caractérisée par le fait qu'à la partie de la portion de serrage (3) qui s'élargit en cône se raccorde une partie cylindrique.

9. Pince selon l'une des revendications précédentes, caractérisée par le fait que les bras (8, 9) sont ondulés selon la direction longitudinale dans la zone de la portion de serrage (3).

10. Pince selon l'une des revendications précédentes, caractérisée par le fait que dans la portion de serrage (3), les bras (8, 9) enserrent entre eux une fente (7) dont la largeur va en croissant de la portion d'extrémité (4) jusqu'aux mors de pincement (10, 11).

11. Pince selon la revendication 10, caractérisée par le fait qu'à l'extrémité de la portion de serrage (3) située du côté des mors de pincement, la fente (7) présente des surfaces d'extrémité orientées vers l'autre bras.

12. Pince selon l'une des revendications précédentes, caractérisée par le fait qu'entre les mors de pincement (10, 11) et la portion de serrage (3), les bras (8, 9) sont cintrés vers l'extérieur en forme d'arc.

13. Pince selon la revendication 12, caractérisée par le fait que les zones (18, 19) des bras (8, 9) cintrés vers l'extérieur en forme d'arc sont formées, à partir d'un épaississement ventru du corps de révolution, par formation d'un méplat des deux côtés et par un élargissement ventru de la fente longitudinale (7).

14. Pince selon l'une des revendications précédentes caractérisée par le fait que dans la zone des mors de pincement (10, 11), la fente longitudinale (7) présente sensiblement une largeur constante, s élargit lors de l'entrée dans la portion de serrage (3) et diminue à nouveau de largeur en direction de la portion d'extrémité (4).

15. Pince selon l'une des revendications précédentes, caractérisée par le fait que la fente longitudinale (7) peut s'obtenir au moyen d'une machine à éroder à fil.
